# EUROPEAN PATENT APPLICATION

(11) **EP 2 186 887 A1**
(43) Date of publication of application: **19.05.2010**
(21) Application number: 10150051.0
(22) Date of filing: 17.11.2006
(51) Int. Cl.: C12N 9/24, C07K 14/435, C12N 9/00

(54) **Glucoamylase variants**

(30) Priority: 18.11.2005 DK 200501611
(62) Divisional of application: 06805576.3
(71) Applicant: Novozymes A/S, 2880 Bagsvaerd (DK)
(72) Inventor: Tams, Jeppe Wegener, 2820, Gentofe (DK); Danielsen, Steffen, 2100 Copenhagen Oe (DK); Friis, Esben Peter, 2730, Herlev (DK)

(57) **Abstract**

The present invention relates to glucoamylase variants with improved properties and methods of utilizing the glucoamylase variants.

## Description

### Technical Field

The present invention relates to glucoamylase variants with improved properties and methods of utilizing the glucoamylase variants.

### Background

Glucoamylase (1,4-alpha-D-glucan glucohydrolase, EC 3.2.1.3) is an enzyme which catalyzes the release of D-glucose from the non-reducing ends of starch or related oligo- and polysaccharide molecules. Glucoamylases are produced by several filamentous fungi and yeasts.

Commercially, the glucoamylase enzyme is used to convert corn starch which is already partially hydrolyzed by an alpha-amylase to glucose. In high fructose corn syrup (HFCS) production the glucose is further converted by glucose isomerase to a mixture composed almost equally of glucose and fructose. This mixture is the commonly used high fructose corn syrup commercialized throughout the world. Most used for high fructose corn syrup are glucoamylases derived from *Talaromyces emersonii* and *Aspergillus niger.*

At the high solids concentrations used commercially for high fructose corn syrup production, glucoamylase synthesizes di-, tri-, and tetra-saccharides from the glucose that is produced by condensation. This occurs because of the slow hydrolysis of alpha-(1-6)-D-glucosidic bonds in starch and the formation of various accumulating condensation products, mainly isomaltose, from D-glucose. Accordingly, the glucose yield in a conventional process does not exceed 95% of theoretical yield. The amount of HFCS produced worldwide by this process is very large and even very small increases in the glucose yield pr. ton of starch are commercially important.

The object of the present invention is to reduce the formation of condensation products of particular glucoamylases, which are obtainable from fungal organisms, in particular strains of the *Talaromyces* genus and *Aspergillus* genus and which themselves had been selected on the basis of their suitable properties in, e.g., starch conversion.

### SUMMARY OF THE INVENTION

The applicants have now found that by introducing certain alterations in specific positions in specific regions of the amino acid sequence of the parent glucoamylase the rate of forming alpha-(1-6) bonds is reduced, and/or the formation of isomaltose is reduced. A reduction of the rate that glucoamylase cleaves and therefore forms alpha-(1-6) bonds relative to the rate it cleaves alpha-(1-4) bonds has practical implications. A glucoamylase that can produce glucose with a significantly reduced amount of by-products would be of great commercial interest, e.g. in production of sweeteners from starch.

The inventors of the present invention have provided a number of variants of a parent glucoamylase, which variants show reduced condensation. By using a glucoamylase variant of the invention in a saccharification process results a syrup with a very high glucose percentage can be produced. The reduced condensation is obtained by mutating, e.g., by substituting and/or deleting and/or inserting selected positions in a parent glucoamylase. This will be described in details below.

Accordingly, in a first aspect the present invention relates to a variant of a parent glucoamylase, which variant glucoamylase have a reduced production of a condensation product when compared to the parent glucoamylase.

Accordingly, in a second aspect the present invention relates to a variant of a parent glucoamylase, comprising an alteration in one of the one of the following regions: the region 248-255, e.g. in one or more of positions 248, 249, 250, 251, 252, 253, 254 and/or 255, the region 309-318, e.g. in one or more of positions 309, 310, 311, 312, 313, 314, 315, 316, 317 and/or 318, and/or the region 409-415, e.g. in one or more of positions 409, 410, 411, 412, 413, 414 and/or 415, wherein (a) the alteration is independently (i) an insertion of an amino acid downstream of the amino acid which occupies the position, (ii) a deletion of the amino acid which occupies the position, or (iii) a substitution of the amino acid which occupies the position with a different amino acid, (b) the variant has glucoamylase activity and (c) each position corresponds to a region or position of the amino acid sequence of the parent glucoamylase having the amino acid sequence of SEQ ID NO: 2 and/or to a region or position in a homologous glucoamylase which displays at least 50% homology with the amino acid sequences shown in SEQ ID NO: 2..

In a third aspect the present invention relates to a DNA construct comprising a DNA sequence encoding a glucoamylase variant according to the first aspect.

In a fouth aspect the present invention relates to a recombinant expression vector which carries a DNA construct according to the second aspect.

In a fifth aspect the present invention relates to a cell which is transformed with a DNA construct according to the second aspect or a vector according to the third aspect.

In a sixth aspect the present invention relates to a cell according the fourth aspect, which is a microorganism, in particular a bacterium or a fungus.

In a seventh aspect the present invention relates to a process for converting starch or partially hydrolyzed starch into a syrup containing dextrose, said process including saccharifying a starch hydrolysate in the presence of a glucoamylase variant according to the first aspect.

In further aspects the present invention relates to a use of a glucoamylase variant of any of the first or second aspects in a starch conversion process, preferably a in a continuous starch conversion process, use in a process for producing oligosaccharides, maltodextrins or glucose syrups, use in a process for producing high fructose corn syrup, use in a process for producing an alcoholic beverage, fuel or drinking ethanol, and/or use in a fermentation process for producing an organic compound.

### DETAILED DISCLOSURE OF THE INVENTION

### Definitions used

### Nomenclature

In the present description and claims, the conventional one-letter and three-letter codes for amino acid residues are used.

For ease of reference, glucoamylase variants of the invention are described by use of the following nomenclature: Original amino acid(s):position(s):substituted amino acid(s).

According to this nomenclature, for instance the substitution of alanine for asparagine in position 30 is shown as: A30N, a deletion of alanine in the same position is shown as: A30*, and insertion of an additional amino acid residue, such as lysine, is shown as: A30AK.

A deletion of a consecutive stretch of amino acid residues, such as amino acid residues 30-33, is indicated as: Δ(A30-N33).

Where a specific glucoamylase contains a "deletion" in comparison with other glucoamylase and an insertion is made in such a position this is indicated as: *36D, for insertion of an aspartic acid in position 36.

Multiple mutations are separated by plus signs: A30N+E34S representing mutations in positions 30 and 34 substituting alanine and glutamic acid for asparagine and serine, respectively. Multiple mutations may also be separated as follows, *i.e.,* meaning the same as the plus sign: A30N/E34S

When one or more alternative amino acid residues may be inserted in a given position it is indicated as: A30N or A30E.

Furthermore, when a position suitable for modification is identified herein without any specific modification being suggested, it is to be understood that any amino acid residue may be substituted for the amino acid residue present in the position. Thus, for instance, when a modification of an alanine in position 30 is mentioned, but not specified, it is to be understood that the alanine may be deleted or substituted for any other amino acid, *i.e.,* any one of: R, N, D, A, C, Q, E, G, H, I, L, K, M, F, P, S, T, W, Y, V.

The terms "polar" (C, T, S, D, N, Y, W, H, K, E, R, Q), "non-polar" (A, C, V, I, L, M, K, F, Y, W, H), "aliphatic" (V, I, L), "aromatic" (F, Y, W, H), "small" (A, C, D, G, N, P, T, S), "tiny" (A, C, G, T, S), "charged" (K, R, D, E) are used for amino acid residues according to the definition in W. R. Taylor in The Classification of Amino Acid Conservation, J. Theor. Biol. 119(1986)205-218. Furthermore the term "six-ring aromatic" is used for amino acid residues containing a non-fused six-membered aromatic ring system (ie. F, Y). Also the term "negative" is used for amino acid residues, which have a negatively charged side chain at neutral pH, (ie. D, E).

In the present context the homology may be determined as the degree of identity between the two sequences indicating a derivation of the first sequence from the second. The homology may suitably be determined by means of computer programs known in the art such as GAP provided in the GCG program package (described above). Thus, Gap GCGv8 may be used with the default scoring matrix for identity and the following default parameters: GAP creation penalty of 5.0 and GAP extension penalty of 0.3, respectively for nucleic acidic sequence comparison, and GAP creation penalty of 3.0 and GAP extension penalty of 0.1, respectively, for protein sequence comparison. GAP uses the method of Needleman and Wunsch, (1970), J.Mol. Biol. 48, p.443-453, to make alignments and to calculate the identity.

A structural alignment between SEQ ID NO1/SEQ ID NO:2 and another glucoamylase may be used to identify equivalent/corresponding positions. One method of obtaining said structural alignment is to use the Pile Up programme from the GCG package using default values of gap penalties, i.e., a gap creation penalty of 3.0 and gap extension penalty of 0.1. Other structural alignment methods include the hydrophobic cluster analysis (Gaboriaud et al., (1987), FEBS LETTERS 224, pp. 149-155) and reverse threading (Huber, T ; Torda, AE, PROTEIN SCIENCE Vol. 7, No. 1 pp. 142-149 (1998).

In the present context, "derived from" is intended not only to indicate an glucoamylase produced or producible by a strain of the organism in question, but also an glucoamylase encoded by a DNA sequence isolated from such strain and produced in a host organism transformed with said DNA sequence. Finally, the term is intended to indicate an glucoamylase, which is encoded by a DNA sequence of synthetic and/or cDNA origin and which has the identifying characteristics of the glucoamylase in question. The term is also intended to indicate that the parent glucoamylase may be a variant of a naturally occurring glucoamylase, i.e. a variant, which is the result of a modification (insertion, substitution, deletion) of one or more amino acid residues of the naturally occurring glucoamylase.

### Glucoamylase variants of the invention

The invention provides variant of a parent glucoamylase, comprising an alteration in one of the following regions: the region 248-255, e.g. in position 248, 249, 250, 251, 252, 253, 254 and/or 255, the region 309-318, e.g. in position 309, 310, 311, 312, 313, 314, 315, 316, 317 and/or 318, and/or the region 409-415, e.g. in position 409, 410, 411, 412, 413, 414 and/or 415, wherein (a) the alteration is independently (i) an insertion of an amino acid downstream of the amino acid which occupies the position, (ii) a deletion of the amino acid which occupies the position, or (iii) a substitution of the amino acid which occupies the position with a different amino acid, (b) the variant has glucoamylase activity and (c) each region and/or position corresponds to a position of the amino acid sequence of the parent glucoamylase having the amino acid sequence of SEQ ID NO: 2 and/or to a region and/or position in a homologous glucoamylase which displays at least 50% homology with the amino acid sequences shown in SEQ ID NO: 2.

Preferred are variants comprising an alteration at one or more of the following positions: 252, 312, 314, 315, 412, wherein each position corresponds to a position of the amino acid sequence of the parent glucoamylase having the amino acid sequence of SEQ ID NO: 2.

Preferred in the position corresponding to L252 in SEQ ID NO:2 is a substitution by any non-polar residue: (A, C, F, G, H, I, K, M, V, W, Y,), more preferably by a non-polar residue which is also a small residue: (V, C, A, G), even more preferably by a non-polar residue which is also a tiny residue: (A, G), and most preferably by residue A.

Preferred in the position corresponding to V312 in SEQ ID NO:2 is a substitution by any polar residue: (C, D, E, H, K, N, Q, R, S, T, W, Y), more preferably by a polar residue which is also a small residue (C, T, S, D, N), even more preferably by a polar residue which is also a tiny residue: (C, T, S) and most preferably by residue S.

Preferred in the position corresponding to Q314 in SEQ ID NO:2 is a substitution by any polar, aliphatic or aromatic residue: (C, D, E, F, H, I, K, L, N, R, S, T, V, W, Y), more preferably by a charged, aliphatic or aromatic residue: (D, E, F, H, I, K, L, R, V, W, Y), more preferably by an aliphatic or aromatic residue (F, H, I, L, V, W, Y), more preferably by an aromatic residue (F, H, W, Y), even more preferably an aromatic residue which is also a six-ring aromatic residue: (F, Y), and most preferably by residue Y.

Preferred in the position corresponding an insertion after Q314 in SEQ ID NO:2 is an insertion by any aliphatic or aromatic residue: (V, I, L, F, Y, W, H), more preferably by an aliphatic residue: (V, I, L) and most preferably insertion of residue I.

Preferred in the position corresponding to G315 in SEQ ID NO:2 is an substitution by any non-polar residue (A, C, F, H, I, K, L, M, V, W, Y), substitution by a polar residue, or by a residue which is not a small residue: (C, D, E, F, H, I, K, L, M, N, Q, R, S, T, W, Y also preferred is a substitution by either a small or a charged residue: (D, E, F, H, I, K, L, M, Q, R, W, Y), more preferably a substitution by a residue which is not a small residue: (E, F, H, I, K, L, M, Q, R, W, Y) or a substitution by a residue which is neither a small nor a positive residue: (E, F, H, I, L, M, Q, W, Y) or a substitution by a residue which is neither a small nor a negative residue: (I, L, M, F, Y, W, H, K, R), or a substitution by a an aromatic or aliphatic residue, which residue is also not a small residue: (F, Y, W, H, I, L), or a substitution by a an aromatic or aliphatic residue, which residue is also neither a small nor a charged residue: (F, Y, W, I, L), or more preferred a substitution by an aromatic residue which is also not a charged residue (F, Y, W), even more preferably an aromatic residue which is also a six-ring aromatic residue: (F, Y), and most preferably by residue Y.

Preferred in the position corresponding to L412 in SEQ ID NO:2 is an substitution by any polar residue (C, D, E, H, K, N, Q, R, S, T, W, Y), more preferably by a charged residue (H, K, R, E, D), yet more preferably by a negative residue (D, E), and most preferably by residue D.

More preferred are variants comprising one or more of the following alterations: substitution to A, F, G or V in position 252; substitution to S in position 312, substitution to E, F, N, R, T, W or Y in position 314; substitution to A, D, F, H, K, L, N, Q, R, S, T or Y in position 315, substitution to D in position 412, or an insertion of amino acid residue I between positions 314 and 315, wherein each position corresponds to a position of the amino acid sequence of the parent glucoamylase having the amino acid sequence of SEQ ID NO: 2.

Even more preferred are variants comprising one or more of the following combinations of substitutions: 314F + 315N, 314W + 315N, 314Y + 315N, 314L + 315A, 314N + 315R, 314R + 315D, 315R + 463T, 315R + 556E, 315L + 529N, wherein each position corresponds to a position of the amino acid sequence of the parent glucoamylase having the amino acid sequence of SEQ ID NO: 2 and in particular variants comprising one or more of the following substitutions: V312S, Q314E, Q314F, Q314N, Q314R, Q314T, Q314W, Q314Y,G315A, G315D, G315F, G315H, G315K, G315L, G315N, G315Q, G315R,G315S, G315T, G315Y,L252A, L252F, L252G, L252V, L412D or an insertion of amino acid residue I between positions 314 and 315 (Q314QI), wherein each position corresponds to a position of the amino acid sequence of the parent glucoamylase having the amino acid sequence of SEQ ID NO: 2.

More preferred are variants comprising one or more of the following combinations of substitutions: Q314F and G315N, Q314W and G315N, Q314Y and G315N, Q314L and G315A, Q314N and G315R, Q314R and G315D, G315R and A463T, G315R and K556E, G315L and D529N, wherein each position corresponds to a position of the amino acid sequence of the parent glucoamylase having the amino acid sequence of SEQ ID NO: 2.

Variants of the invention may have at least 20%, preferably at least 40%, more preferably at least 60%, even more preferably at least 80%, even more preferably at least 90%, and most preferably at least 100% of the glucoamylase activity of the mature glucoamylase of SEQ ID NO: 2.

Variants of the invention may have a condensation which have been reduced with at least 5%, preferably at least 10%, more preferably at least 15%, even more preferably at least 20%, yet preferably at least 25%, and most preferably at least 30% relative to the glucoamylase of the parent glucoamylase of SEQ ID NO: 2.

### Parent Glucoamylases

Parent glucoamylase contemplated according to the present invention include wild-type glucoamylases, fungal glucoamylases, in particular fungal glucoamylases obtainable from an *Talaromyces,* in particular *T. emersonii* disclosed in WO 99/28448 (See SEQ ID NO: 7 of WO 99/28448) and in SEQ ID NO:2 herein. In another embodiment the glucoamylase backbone is derived from *an Aspergillus* strain, such as an *Aspergillus niger* or *Aspergillus awamori* glucoamylases and variants or mutants thereof, homologous glucoamylases, and further glucoamylases being structurally and/or functionally similar thereto.

Preferably, the parent glucoamylase comprises one or more specific amino acid residues selected from the list comprising; L amino residue in position 252, Q amino residue in position 314, G amino acid residue in position 315 and L amino residue in position 412.

Preferably, the parent glucoamylase comprises the amino acid sequences of SEQ ID NO: 2; or allelic variants thereof; or a fragment thereof that has glucoamylase activity.

A fragment of SEQ ID NO: 2 is a polypeptide which has one or more amino acids deleted from the amino and/or carboxyl terminus of this amino acid sequence. An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

A suitable parent glucoamylase may be a glucoamylase having an amino acid sequence which has a degree of identity to the amino acid sequence of SEQ ID NO: 2 of at least 50%, preferably at least 60%, more preferably at least about 70%, yet more preferably at least 80%, even more preferably at least 90%, most preferably at least 95%, and most preferably at least 98% (i.e. homologous parent glucoamylases).

The amino acid sequences of homologous parent glucoamylases may differ from the amino acid sequence of SEQ ID NO: 2 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

In a embodiment, the isolated parent glucoamylase is encoded by a nucleic acid sequence which hybridises under very low stringency conditions, preferably low stringency conditions, more preferably medium stringency conditions, more preferably medium-high stringency conditions, even more preferably high stringency conditions, and most preferably very high stringency conditions with a nucleic acid probe which hybridises under the same conditions with (i) the nucleic acid sequence of SEQ ID NO: 1, (ii) the cDNA sequence of SEQ ID NO:1, (iii) a sub-sequence of (i) or (ii), or (iv) a complementary strand of (i), (ii), or (iii).

In another embodiment, the isolated parent glucoamylase is encoded by a nucleic acid sequence which hybridises under very low stringency conditions, preferably low stringency conditions, more preferably medium stringency conditions, more preferably medium-high stringency conditions, even more preferably high stringency conditions, and most preferably very high stringency conditions with (i) the nucleic acid sequence of SEQ ID NO: 1, (ii) the cDNA sequence of SEQ ID NO:1, (iii) a sub-sequence of (i) or (ii), or (iv) a complementary strand of (i), (ii), or (iii).

Suitable conditions for testing hybridization involve presoaking in 5xSSC and prehybridizing for 1 hour at ∼40°C in a solution of 20% formamide, 5xDenhardt's solution, 50mM sodium phosphate, pH 6.8, and 50mg of denatured sonicated calf thymus DNA, followed by hybridization in the same solution supplemented with 100mM ATP for 18 hours at ∼40°C, followed by three times washing of the filter in 2xSSC, 0.2% SDS at 40°C for 30 minutes (low stringency), preferred at 50°C (medium stringency), more preferably at 65°C (high stringency), even more preferably at ∼75°C (very high stringency). (J. Sambrook, E.F. Fritsch, and T. Maniatus, 1989, Molecular Cloning, A Laboratory Manual, 2d edition, Cold Spring Harbor, New York). The sub-sequence of SEQ ID NO: 1 may be at least 100 nucleotides or preferably at least 200 nucleotides. Moreover, the sub-sequence may encode a polypeptide fragment, which has glucoamylase activity. The parent polypeptides may also be allelic variants or fragments of the polypeptides that have glucoamylase activity.

The nucleic acid sequence of SEQ ID NO: 1 or a subsequence thereof, as well as the amino acid sequence of SEQ ID NO: 2, or a fragment thereof, may be used to design a nucleic acid probe to identify and clone DNA encoding polypeptides having glucoamylase activity, from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with ³²P, ³H, ³⁵S, biotin, or avidin).

Thus, a genomic DNA or cDNA library prepared from such other organisms may be screened for DNA, which hybridizes with the probes described above and which encodes a polypeptide having glucoamylase. Genomic or other DNA from such other organisms may be separated by agarose or polyacrylamide gel electrophoresis, or other separation techniques. DNA from the libraries or the separated DNA may be transferred to and immobilised on nitrocellulose or other suitable carrier material. In order to identify a clone or DNA which is homologous with SEQ ID NO: 1, or sub-sequences thereof, the carrier material is used in a Southern blot. For purposes of the present invention, hybridisation indicates that the nucleic acid sequence hybridises to a nucleic acid probe corresponding to the nucleic acid sequence shown in SEQ ID NO: 1 its complementary strand, or a sub-sequence thereof, under very low to very high stringency conditions. Molecules to which the nucleic acid probe hybridises under these conditions are detected using X-ray film.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

Contemplated parent glucoamylases have at least 20%, preferably at least 40%, more preferably at least 60%, even more preferably at least 80%, even more preferably at least 90%, and most preferably at least 100% of the glucoamylase activity of the mature glucoamylase of SEQ ID NO: 2.

### Cloning a DNA sequence encoding a parent glucoamylase

The DNA sequence encoding a parent glucoamylase may be isolated from any cell or microorganism producing the glucoamylase in question, using various methods well known in the art. First, a genomic DNA and/or cDNA library should be constructed using chromosomal DNA or messenger RNA from the organism that produces the glucoamylase to be studied. Then, if the amino acid sequence of the glucoamylase is known, labeled oligonucleotide probes may be synthesized and used to identify glucoamylase-encoding clones from a genomic library prepared from the organism in question. Alternatively, a labelled oligonucleotide probe containing sequences homologous to another known glucoamylase gene could be used as a probe to identify glucoamylase-encoding clones, using hybridization and washing conditions of very low to very high stringency. This is described above.

Yet another method for identifying glucoamylase-encoding clones would involve inserting fragments of genomic DNA into an expression vector, such as a plasmid, transforming glucoamylase-negative bacteria with the resulting genomic DNA library, and then plating the transformed bacteria onto agar containing a substrate for glucoamylase (*i.e.,* maltose), thereby allowing clones expressing the glucoamylase to be identified.

Alternatively, the DNA sequence encoding the enzyme may be prepared synthetically by established standard methods, *e.g*. the phosphoroamidite method described S.L. Beaucage and M.H. Caruthers, (1981), Tetrahedron Letters 22, p. 1859-1869, or the method described by Matthes et al., (1984), EMBO J. 3, p. 801-805. In the phosphoroamidite method, oligonucleotides are synthesized, *e.g*., in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al., (1988), Science 239, 1988, pp. 487-491.

### Site-directed mutagenesis

Once a glucoamylase-encoding DNA sequence has been isolated, and desirable sites for mutation identified, mutations may be introduced using synthetic oligonucleotides. These oligonucleotides contain nucleotide sequences flanking the desired mutation sites. In a specific method, a single-stranded gap of DNA, the glucoamylase-encoding sequence, is created in a vector carrying the glucoamylase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al., (1984), Biotechnology 2, p. 646-639. US 4,760,025 disclose the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method for introducing mutations into glucoamylase-encoding DNA sequences is described in Nelson and Long, (1989), Analytical Biochemistry 180, p. 147-151. It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

Further, Sierks. et al., (1989) "Site-directed mutagenesis at the active site Trp120 of Aspergillus awamori glucoamylase. Protein Eng., 2, 621-625; Sierks et al., (1990), "Determination of Aspergillus awamori glucoamylase catalytic mechanism by site-directed mutagenesis at active site Asp176, Glu179, and Glu180". Protein Eng. vol. 3, 193-198; also describes site-directed mutagenesis in an *Aspergillus* glucoamylase.

### Expression of glucoamylase variants

According to the invention, a DNA sequence encoding a glucoamylase variant produced by methods described above, or by any alternative methods known in the art, can be expressed, in enzyme form, using an expression vector which typically includes control sequences encoding a promoter, operator, ribosome binding site, translation initiation signal, and, optionally, a repressor gene or various activator genes.

### Expression vector

The recombinant expression vector carrying the DNA sequence encoding a glucoamylase variant of the invention may be any vector, which may conveniently be subjected to recombinant DNA procedures, and the choice of vector will often depend on the host cell into which it is to be introduced. The vector may be one which, when introduced into a host cell, is integrated into the host cell genome and replicated together with the chromosome(s) into which it has been integrated. Examples of suitable expression vectors include pMT838.

### Promoter

In the vector, the DNA sequence should be operably connected to a suitable promoter sequence. The promoter may be any DNA sequence, which shows transcriptional activity in the host cell of choice and may be derived from genes encoding proteins either homologous or heterologous to the host cell.

Examples of suitable promoters for directing the transcription of the DNA sequence encoding a glucoamylase variant of the invention, especially in a bacterial host, are the promoter of the *lac* operon of *E.coli,* the *Streptomyces coelicolor* agarase gene *dag*A promoters, the promoters of the *Bacillus licheniformis* alpha-amylase gene (*amyL*), the promoters of the Ba*cillus stearothermophilus* maltogenic amylase gene (*amyM*), the promoters of the *Bacillus amyloliquefaciens* alpha-amylase (*amyQ*), the promoters of the *Bacillus subtilis* xylA and xyIB genes etc. For transcription in a fungal host, examples of useful promoters are those derived from the gene encoding *A. oryzae* TAKA amylase, the TPI (triose phosphate isomerase) promoter from S. *cerevisiae* (Alber et al. (1982), J. Mol. Appl. Genet 1, p. 419-434, *Rhizomucor miehei* aspartic proteinase, *A. niger* neutral alpha-amylase, *A. niger* acid stable alpha-amylase, *A. niger* glucoamylase, *Rhizomucor miehei* lipase, *A. oryzae* alkaline protease, *A. oryzae* triose phosphate isomerase or *A*. *nidulans* acetamidase.

### Expression vector

The expression vector of the invention may also comprise a suitable transcription terminator and, in eukaryotes, polyadenylation sequences operably connected to the DNA sequence encoding the glucoamylase variant of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the *dal* genes from *B. subtilis* or *B. licheniformis,* or one which confers antibiotic resistance such as ampicillin, kanamycin, chloramphenicol or tetracyclin resistance. Furthermore, the vector may comprise *Aspergillus* selection markers such as amdS, argB, niaD and sC, a marker giving rise to hygromycin resistance, or the selection may be accomplished by co-transformation, *e.g*., as described in WO 91/17243.

The procedures used to ligate the DNA construct of the invention encoding a glucoamylase variant, the promoter, terminator and other elements, respectively, and to insert them into suitable vectors containing the information necessary for replication, are well known to persons skilled in the art (cf., for instance, Sambrook et al., Molecular Cloning: A Laboratory Manual, 2nd Ed., Cold Spring Harbor, 1989).

### Host Cells

The cell of the invention, either comprising a DNA construct or an expression vector of the invention as defined above, is advantageously used as a host cell in the recombinant production of a glucoamylase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, *e.g*. by homologous or heterologous recombination. Alternatively, the cell may be transformed with an expression vector as described above in connection with the different types of host cells.

The cell of the invention may be a cell of a higher organism such as a mammal, an insect or a plant, but is preferably a microbial cell, e.g., a bacterial or a fungal (including yeast) cell.

Examples of suitable bacteria are Gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis,* or *Streptomyces lividans* or *Streptomyces murinus,* or gram-negative bacteria such as *E.coli.* The transformation of the bacteria may, for instance, be effected by protoplast transformation or by using competent cells in a manner known *per se.*

The yeast organism may favorably be selected from a species of *Saccharomyces* or *Schizosaccharomyces,* e.g*., Saccharomyces cerevisiae.*

The host cell may also be a filamentous fungus,*e.g*., a strain belonging to a species of *Aspergillus,* most preferably *Aspergillus oryzae* or *Aspergillus niger, or* a strain of *Fusarium,* such as a strain of *Fusarium oxysporium, Fusarium graminearum* (in the perfect state named *Gribberella zeae,* previously *Sphaeria zeae, synonym with Gibberella roseum and Gibberella roseum* f. sp. *cerealis)*, or *Fusarium sulphureum* (in the prefect state named *Gibberella puricaris,* synonym with *Fusarium trichothecioides, Fusarium bactridioides, Fusarium sambucium, Fusarium roseum,* and *Fusarium roseum var. graminearum), Fusarium cerealis* (synonym with *Fusarium crokkwellnse), or Fusarium venenatum.*

In a preferred embodiment of the invention the host cell is a protease deficient or protease minus strain.

This may for instance be the protease deficient strain *Aspergillus oryzae* JaL 125 having the alkaline protease gene named "alp" deleted. This strain is described in WO 97/35956 (Novo Nordisk), or EP patent no. 429,490.

Filamentous fungi cells may be transformed by a process involving protoplast formation and transformation of the protoplasts followed by regeneration of the cell wall in a manner known per se. The use of *Aspergillus* as a host micro-organism is described in EP 238,023 (Novo Nordisk A/S), the contents of which are hereby incorporated by reference.

### Expression of the glucoamylase variants in plants

A DNA sequence encoding a polypeptide of interest, such as a glucoamylase of the present invention, may be transformed and expressed in transgenic plants as described below.

The transgenic plant can be dicotyledonous or monocotyledonous, for short a dicot or a monocot. Examples of monocot plants are grasses, such as meadow grass (blue grass, Poa), forage grass such as *Festuca, Lolium,* temperate grass, such as *Agrostis,* and cereals, *e.g*., wheat, oats, rye, barley, rice, sorghum and maize (corn).

Examples of dicot plants are tobacco, legumes, such as lupins, potato, sugar beet, pea, bean and soybean, and cruciferous plants (family *Brassicaceae),* such as cauliflower, oil seed rape and the closely related model organism *Arabidopsis thaliana.*

Examples of plant parts are stem, callus, leaves, root, fruits, seeds, and tubers as well as the individual tissues comprising these parts, *e.g*., epidermis, mesophyll, parenchyme, vascular tissues, meristems. In the present context, also specific plant cell compartments, such as chloroplast, apoplast, mitochondria, vacuole, peroxisomes and cytoplasm are considered to be a plant part. Furthermore, any plant cell, whatever the tissue origin, is considered to be a plant part. Likewise, plant parts such as specific tissues and cells isolated to facilitate the utilisation of the invention are also considered plant parts, *e.g*., embryos, endosperms, aleurone and seeds coats.

Also included within the scope of the invention are the progeny of such plants, plant parts and plant cells.

The transgenic plant or plant cell expressing the polypeptide of interest may be constructed in accordance with methods known in the art. In short the plant or plant cell is constructed by incorporating one or more expression constructs encoding the polypeptide of interest into the plant host genome and propagating the resulting modified plant or plant cell into a transgenic plant or plant cell.

Conveniently, the expression construct is a DNA construct which comprises a gene encoding the polypeptide of interest in operable association with appropriate regulatory sequences required for expression of the gene in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences is determined, *e.g*., on the basis of when, where and how the enzyme is desired to be expressed. For instance, the expression of the gene encoding the enzyme of the invention may be constitutive or inducible, or may be developmental, stage or tissue specific, and the gene product may be targeted to a specific cell compartment, tissue or plant part such as seeds or leaves. Regulatory sequences are, *e.g*., described by Tague et al, Plant Phys., 86, 506, 1988.

For constitutive expression the 35S-CaMV, the maize ubiquitin 1 and the rice actin 1 promoter may be used (Franck et al. 1980. Cell 21: 285-294, Christensen AH, Sharrock RA and Quail 1992. Maize polyubiquitin genes: structure, thermal perturbation of expression and transcript splicing, and promoter activity following transfer to protoplasts by electroporation. Plant Mo. Biol. 18, 675-689.; Zhang W, McElroy D. and Wu R 1991, Analysis of rice Act1 5' region activity in transgenic rice plants. Plant Cell 3, 1155-1165). Organ-specific promoters may, *e.g*., be a promoter from storage sink tissues such as seeds, potato tubers, and fruits (Edwards & Coruzzi, 1990. Annu. Rev. Genet. 24: 275-303), or from metabolic sink tissues such as meristems (Ito et al., 1994, Plant Mol. Biol. 24: 863-878), a seed specific promoter such as the glutelin, prolamin, globulin or albumin promoter from rice (Wu et al., Plant and Cell Physiology Vol. 39, No. 8 pp. 885-889 (1998)), a *Vicia faba* promoter from the legumin B4 and the unknown seed protein gene from *Vicia faba* described by Conrad U. et al, Journal of Plant Physiology Vol. 152, No. 6, pp. 708-711 (1998), a promoter from a seed oil body protein (Chen et al., Plant and Cell Physiology, Vol. 39, No. 9, pp. 935-941 (1998), the storage protein napA promoter from *Brassica napus,* or any other seed specific promoter known in the art, *e.g*., as described in WO 91/14772. Furthermore, the promoter may be a leaf specific promoter such as the rbcs promoter from rice or tomato (Kyozuka et al., Plant Physiology, Vol. 102, No. 3, pp. 991-1000 (1993), the chlorella virus adenine methyltransferase gene promoter (Mitra, A. and Higgins, DW, Plant Molecular Biology, Vol. 26, No. 1, pp. 85-93 (1994), or the aldP gene promoter from rice (Kagaya et al., Molecular and General Genetics, Vol. 248, No. 6, pp. 668-674 (1995), or a wound inducible promoter such as the potato pin2 promoter (Xu et al, Plant Molecular Biology, Vol. 22, No. 4, pp. 573-588 (1993). Likewise, the promoter may inducible by abiotic treatments such as temperature, drought or alterations in salinity or induced by exogenously applied substances that activate the promoter, *e.g*., ethanol, oestrogens, plant hormones like ethylene, abscisic acid and gibberellic acid and heavy metals.

A promoter enhancer element may be used to achieve higher expression of the enzyme in the plant. For instance, the promoter enhancer element may be an intron which is placed between the promoter and the nucleotide sequence encoding the enzyme. For instance, Xu et al. *op cit* disclose the use of the first intron of the rice actin 1 gene to enhance expression.

The selectable marker gene and any other parts of the expression construct may be chosen from those available in the art.

The DNA construct is incorporated into the plant genome according to conventional techniques known in the art, including Agrobacterium-mediated transformation, virus-mediated transformation, micro injection, particle bombardment, biolistic transformation, and electroporation (Gasser et al, Science, 244, 1293; Potrykus, Bio/Techn. 8, 535, 1990; Shimamoto et al, Nature, 338, 274, 1989).

Presently, *Agrobacterium tumefaciens* mediated gene transfer is the method of choice for generating transgenic dicots (for review Hooykas & Schilperoort, 1992, Plant Mol. Biol., 19: 15-38), and can also be used for transforming monocots, although other transformation methods often are used for these plants. Presently, the method of choice for generating transgenic monocots supplementing the *Agrobacterium* approach is particle bombardment (microscopic gold or tungsten particles coated with the transforming DNA) of embryonic calli or developing embryos (Christou, 1992, Plant J., 2: 275-281; Shimamoto, 1994, Curr. Opin. Biotechnol., 5: 158-162; Vasil et al., 1992, Bio/Technology 10: 667-674). An alternative method for transformation of monocots is based on protoplast transformation as described by Omirulleh S, et al., Plant Molecular Biology, Vol. 21, No. 3, pp. 415-428 (1993).

Following transformation, the transformants having incorporated the expression construct are selected and regenerated into whole plants according to methods well-known in the art. Often the transformation procedure is designed for the selective elimination of selection genes either during regeneration or in the following generations by using, *e.g*., co-transformation with two separate T-DNA constructs or site specific excision of the selection gene by a specific recombinase.

### Method of producing glucoamylase variants

The present invention also relates to a method of producing a glucoamylase variant of the invention, which method comprises cultivating a host cell under conditions conducive to the production of the variant and recovering the variant from the cells and/or culture medium.

The medium used to cultivate the cells may be any conventional medium suitable for growing the host cell in question and obtaining expression of the glucoamylase variant of the invention. Suitable media are available from commercial suppliers or may be prepared according to published recipes (*e.g*. as described in catalogues of the American Type Culture Collection).

The glucoamylase variant secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### Uses of the glucoamylase variants

Variants of the invention may be used in a starch conversion process, e.g. any starch degradation process wherein starch is degraded to e.g. glucose, e.g. such as in production of sweeteners, or in fermentation process for producing an organic compound, e.g. such as ethanol, citric acid, ascorbic acid, lysine, citric acid, monosodium glutamate, gluconic acid, sodium gluconate, calcium gluconate, potassium gluconate, glucono delta lactone, sodium erythorbate, itaconic acid, lactic acid, gluconic acid; ketones; amino acids, glutamic acid (sodium monoglutaminate), penicillin, tetracyclin; enzymes; vitamins, such as riboflavin, B12, beta-carotene or hormones.

Conventional starch-conversion processes, such as liquefaction and saccharification processes are described, e.g., in US Patent No. 3,912,590 and EP patent publications Nos. 252,730 and 63,909, hereby incorporated by reference.

Variants of the invention are particularly useful for starch conversion, e.g. such as in production of the sweetener high fructose corn syrup (HFCS).

Variants of the invention may be used in mashing and/or fermentation processes for producing an alcoholic beverage, fuel or drinking ethanol.

### Starch conversion

The present invention provides a method of using glucoamylase variants of the invention for producing glucose and the like from starch. Generally, the method includes the steps of partially hydrolyzing precursor starch in the presence of alpha-amylase and then further hydrolyzing the release of D-glucose from the non-reducing ends of the starch or related oligo- and polysaccharide molecules in the presence of glucoamylase by cleaving alpha-(1-4) and alpha-(1-6) glucosidic bonds.

The partial hydrolysis of the precursor starch utilizing alpha-amylase provides an initial breakdown of the starch molecules by hydrolyzing internal alpha-(1-4)-linkages. In commercial applications, the initial hydrolysis using alpha-amylase is run at a temperature of approximately 105°C. A very high starch concentration is processed, usually 30% to 40% solids. The initial hydrolysis is usually carried out for five minutes at this elevated temperature. The partially hydrolyzed starch can then be transferred to a second tank and incubated for approximately one hour at a temperature of 85° to 90°C to derive a dextrose equivalent (D.E.) of 10 to 15.

The step of further hydrolyzing the release of D-glucose from the non-reducing ends of the starch or related oligo- and polysaccharides molecules in the presence of glucoamylase is normally carried out in a separate tank at a reduced temperature between 30° and 60°C. Preferably the temperature of the substrate liquid is dropped to between 55°C and 60°C. The pH of the solution is dropped from 6 to 6.5 to a range between 3 and 5.5. Preferably, the pH of the solution is 4 to 4.5. The glucoamylase is added to the solution and the reaction is carried out for 24-72 hours, preferably 36-48 hours.

Examples of saccharification processes wherein the glucoamylase variants of the invention may be used include the processes described in JP 3-224493; JP 1-191693; JP 62-272987; and EP 452,238.

The glucoamylase variant(s) of the invention may be used in the present inventive process in combination with an enzyme that hydrolyzes only alpha-(1-6)-glucosidic bonds in molecules with at least four glucosyl residues. Preferentially, the glucoamylase variant of the invention can be used in combination with pullulanase or isoamylase. The use of isoamylase and pullulanase for debranching, the molecular properties of the enzymes, and the potential use of the enzymes with glucoamylase is set forth in G.M.A. van Beynum et al., Starch Conversion Technology, Marcel Dekker, New York, 1985, 101-142.

The invention also relates to the use of a glucoamylase variant of the invention in a starch conversion process, preferably in a continuous saccharification step.

The glucoamylase variants of the invention may also be used in immobilised form. This is suitable and often used for producing maltodextrins or glucose syrups or speciality syrups, such as maltose syrups, and further for the raffinate stream of oligosaccharides in connection with the production of fructose syrups.

When the desired final sugar product is, *e.g*., high fructose syrup the dextrose syrup may be converted into fructose. After the saccharification process the pH is increased to a value in the range of 6-8, preferably pH 7.5, and the calcium is removed by ion exchange. The dextrose syrup is then converted into high fructose syrup using, e.g., an immmobilized glucose isomerase (such as Sweetzyme™ IT).

### Fermentation process

Maltose and/or glucose may be fermented to an ethanol product or other fermentation products, such as citric acid, monosodium glutamate, gluconic acid, sodium gluconate, calcium gluconate, potassium gluconate, glucono delta lactone, or sodium erythorbate, itaconic acid, lactic acid, gluconic acid; ketones; amino acids, glutamic acid (sodium monoglutaminate), penicillin, tetracyclin; enzymes; vitamins, such as riboflavin, B12, beta-carotene or hormones.

In general a fermentation process based on whole grain, e.g. an ethanol process, can be separated into 4 main steps, - milling, - liquefaction, - saccharification, - fermentation

The grain is milled in order to open up the structure and allowing for further processing. Two processes are used wet or dry milling. In dry milling the whole kernel is milled and used in the remaining part of the process. Wet milling gives a very good separation of germ and meal (starch granules and protein) and is with a few exceptions applied at locations where there is a parallel production of syrups.

In the liquefaction process the starch granules are solubilized by hydrolysis to maltodextrins mostly of a DP higher than 4. The hydrolysis may be carried out by acid treatment or enzymatically by alpha-amylase. Acid hydrolysis is used on a limited basis. The raw material can be milled whole grain or a side stream from starch processing.

Enzymatic liquefaction is typically carried out as a three-step hot slurry process. The slurry is heated to between 60-95°C, preferably 80-85°C, and the enzyme(s) is (are) added. Then the slurry is jet-cooked at between 95-140°C, preferably 105-125°C to gelatinize the starch, cooled to 60-95°C and more enzyme(s) is (are) added to obtain the final hydrolysis. The liquefaction process is carried out at pH 4.5-6.5, typically at a pH between 5 and 6. Milled and liquefied grain is also known as mash.

To produce low molecular sugars DP₁₋₃ that can be metabolized by yeast, the maltodextrin from the liquefaction must be further hydrolyzed. The hydrolysis is typically done enzymatically by glucoamylases, alternatively alpha-glucosidases or acid alpha-amylases can be used. A full saccharification step may last up to 72 hours, however, it is common only to do a pre-saccharification of typically 40-90 minutes and then complete saccharification during fermentation (SSF). Saccharification is typically carried out at temperatures from 30-65°C, typically around 60°C, and at pH 4.5.

Yeast typically from *Saccharomyces* spp. is added to the mash and the fermentation is ongoing for 24-96 hours, such as typically 35-60 hours. The temperature is between 26-34°C, typically at about 32°C, and the pH is from pH 3-6, preferably around pH 4-5.

Note that the most widely used process is a simultaneous saccharification and fermentation (SSF) process where there is no holding stage for the saccharification, meaning that yeast and enzyme is added together. When doing SSF it is common to introduce a pre-saccharification step at a temperature above 50°C, just prior to the fermentation.

The liquefaction and saccharification may also be performed without gelatinizing the starch in a so called raw starch hydrolysis process, e.g. such as described in WO 2004/080923, WO 2004/081193 or WO 2003/66826. A raw starch hydrolysis process is preferably performed as an SSF process.

Following the fermentation the mash is distilled to extract the ethanol. The ethanol obtained according to the process of the invention may be used as, e.g., fuel ethanol; drinking ethanol, i.e., potable neutral spirits; or industrial ethanol.

Left over from the fermentation is the spend grain, which is typically used for animal feed either in liquid form or dried.

Further details on how to carry out liquefaction, saccharification, fermentation, distillation, and recovering of ethanol are well known to the skilled person.

### METHODS

### Glucoamylase activity (AGU)

Glucoamylase activity may be measured in AGU units. One AGU is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| AMG incubation: | |
|---|---|
| Substrate: | maltose 23.2 mM |
| Buffer: | acetate 0.1 M |
| pH: | 4.30 ± 0.05 |
| Incubation temperature: | 37°C ± 1 |
| Reaction time: | 5 minutes |
| Enzyme working range: | 0.5-4.0 AGU/mL |

| Color reaction: | |
|---|---|
| GlucDH: | 430 U/L |
| Mutarotase: | 9 U/L |
| NAD: | 0.21 mM |
| Buffer: | phosphate 0.12 M; 0.15 M NaCl |
| pH: | 7.60 ± 0.05 |
| Incubation temperature: | 37°C ± 1 |
| Reaction time: | 5 minutes |
| Wavelength: | 340 nm |

A folder (EB-SM-0131.02/01) describing this analytical method in more detail is available on request from Novozymes A/S, Denmark, which folder is hereby included by reference.

### EXAMPLES

### Example 1

The parent glucoamylase from *Talaromyces emersonii* (SEQ ID NO:2) and selected variants comprising specific substitutions were tested in a saccharification assay with 30% DS of a DE11 liquefied starch, at pH 4.3 and 60°C. The DE11 liquefied starch was prepared from a 33% DS Cerestar corn starch with 15 ppm Ca++ at pH 5.2 and liquefied with Termamyl Supra (120 KNU(T)/g). The assay was performed in duplicate. The assay was performed without and with additional enzymes; 0.012 AFAU/g DS acid alpha-amylase from *Aspergillus niger* and 0.2NPUN/g DS pullulanase from *Bacillus amyloderamificans.*

| Table 1. Saccharification with 0.04 mg glucoamylase enzyme/g DS. LSD is +-0.2 for DP1 and +-0.1 for DP2 | | | | | | | | |
|---|---|---|---|---|---|---|---|---|
| **Hrs** | **L252A** | | **G315F** | | **G315Y** | | **SEQ ID NO:2** | |
| | **DP1** | **DP2** | **DP1** | **DP2** | **DP1** | **DP2** | **DP1** | **DP2** |
| **24** | 82,8 | 1,2 | 81,4 | 1,0 | 82,2 | 1,0 | 81,4 | 1,4 |
| **48** | 87,8 | 1,7 | 86,8 | 1,3 | 87,5 | 1,4 | 86,5 | 2,0 |
| **72** | 89,6 | 2,4 | 89,1 | 1,7 | 89,8 | 1,7 | 88,4 | 2,8 |
| **96** | 90,6 | 2,9 | 90,3 | 2,0 | 90,8 | 2,1 | 89,1 | 3,5 |

| Table 2. Saccharification with 0.04 mg glucoamylase enzyme/g DS + 0.012 AFAU/g DS + 0.2NPUN/g DS. LSD is +-0.2 for DP1 and +-0.1 for DP2 | | | | | | | | |
|---|---|---|---|---|---|---|---|---|
| **Hrs** | **L252A** | | **G315F** | | **G315Y** | | **SEQ ID NO:2** | |
| | **DP1** | **DP2** | **DP1** | **DP2** | **DP1** | **DP2** | **DP1** | **DP2** |
| **24** | 94,4 | 2,2 | 92,2 | 2,2 | 93,3 | 2,0 | 94,1 | 2,4 |
| **48** | 96,6 | 2,2 | 96,7 | 1,7 | 96,9 | 1,7 | 96,3 | 2,5 |
| **72** | 96,3 | 2,7 | 96,8 | 2,1 | 96,8 | 2,1 | 95,9 | 3,2 |
| **96** | 96,0 | 3,2 | 96,7 | 2,3 | 96,7 | 2,5 | 95,4 | 3,9 |

## Claims

1. A variant of a parent glucoamylase, comprising an alteration in position 315 , wherein
(a) the alteration is,
(i) an insertion of an amino acid downstream of the amino acid which occupies the position,
(ii) a deletion of the amino acid which occupies the position, or
(iii) a substitution of the amino acid which occupies the position with a different amino acid,
(b) the variant has glucoamylase activity and
(c) the position corresponds to a position of the amino acid sequence of the parent glucoamylase having the amino acid sequence of SEQ ID NO: 2 and/or to a position in a homologous glucoamylase which displays at least 50% homology with the amino acid sequences shown in SEQ ID NO: 2.

2. The variant of claim 1, which variant comprises one or more of the following alterations: substitution to F, Y, A, D, H, K, L, N, Q, R, S, or T in position 315.

3. The variant of any of claims 1 or 2, which variant comprises one or more of the following substitutions: G315F, G315Y, G315A, G315D, G315H, G315K, G315L, G315N, G315Q, G315R, G315S, G315T,), wherein the position corresponds to a position of the amino acid sequence of the parent glucoamylase having the amino acid sequence of SEQ ID NO: 2 and/or to a position in a homologous glucoamylase which displays at least 50% homology with the amino acid sequences shown in SEQ ID NO: 2.

4. The variant of any of claims 1 to 3, which variant comprises one or more of the following combinations of substitutions: 314F + 315N, 314W + 315N, 314Y + 315N, 314L + 315A, 314N + 315R, 314R + 315D, 315R + 463T, 315R + 556E, 315L + 529N, wherein each position corresponds to a position of the amino acid sequence of the parent glucoamylase having the amino acid sequence of SEQ ID NO: 2 and/or to a position in a homologous glucoamylase which displays at least 50% homology with the amino acid sequences shown in SEQ ID NO: 2.

5. The variant of any of claims 1 to 4, which variant comprises one or more of the following combinations of substitutions: Q314F + G315N, Q314W + G315N, Q314Y + G315N, Q314L + G315A, Q314N + G315R, Q314R + G315D, G315R + A463T, , G315L + D529N, wherein each position corresponds to a position of the amino acid sequence of the parent glucoamylase having the amino acid sequence of SEQ ID NO: 2 and/or to a position in a homologous glucoamylase which displays at least 50% homology with the amino acid sequences shown in SEQ ID NO: 2.

6. The variant of any of claims 1 to 5, wherein the parent glucoamylase has an amino acid sequence which has a degree of identity to the amino acid sequence of SEQ ID NO: 2 of at least 60%, preferably at least 60%, more preferably at least about 70%, yet more preferably at least 80%, even more preferably at least 90%, most preferably at least 95%, and most preferably at least 98%.

7. The variant of any of claims 1 to 6, wherein the parent glucoamylase is obtained from the genus *Talaromyces,* in particular *Talaromyces emersonii.*

8. The variant of any of claims 1 to 7, wherein the variant has reduced condensation when compared with the parent glucoamylase.

9. A DNA construct comprising a DNA sequence encoding a glucoamylase variant according to any one of claims 1 to 8.

10. A cell which is transformed with a DNA construct according to claim 9.

11. A cell according to claim 10, which is a microorganism, in particular a bacterium or a fungus.

12. A process for converting starch or partially hydrolyzed starch into a syrup containing dextrose, said process including saccharifying a starch hydrolysate in the presence of a glucoamylase variant according to any of claims 1 to 8.

13. Use of a glucoamylase variant according to any of claims 1 to 8 in a process for producing oligosaccharides, maltodextrins or glucose syrups.

14. Use of a glucoamylase variant according to any one of claim 1 to 8 in a process for producing high fructose corn syrup.

15. Use of a glucoamylase variant according to any one of claim 1 to 8 in a process for producing an alcoholic beverage, fuel or drinking ethanol.


SEQUENCE LISTING

<110>  Novozymes A/S

<120>  T-AMG Variants

<130>  10870.204-WO

<160>  1

<170>  PatentIn version 3.3

<210>  1
<211>  591
<212>  PRT
<213>  Talaromyces emersonii


<220>
<221>  mat_peptide
<222>  (1)..(591)

<400>  1

Ala Thr Gly Ser Leu Asp Ser Phe Leu Ala Thr Glu Thr Pro Ile Ala
1               5                   10                  15


Leu Gln Gly Val Leu Asn Asn Ile Gly Pro Asn Gly Ala Asp Val Ala
            20                  25                  30


Gly Ala Ser Ala Gly Ile Val Val Ala Ser Pro Ser Arg Ser Asp Pro
        35                  40                  45


Asn Tyr Phe Tyr Ser Trp Thr Arg Asp Ala Ala Leu Thr Ala Lys Tyr
    50                  55                  60


Leu Val Asp Ala Phe Ile Ala Gly Asn Lys Asp Leu Glu Gln Thr Ile
65                  70                  75                  80


Gln Gln Tyr Ile Ser Ala Gln Ala Lys Val Gln Thr Ile Ser Asn Pro
                85                  90                  95


Ser Gly Asp Leu Ser Thr Gly Gly Leu Gly Glu Pro Lys Phe Asn Val
            100                 105                 110


Asn Glu Thr Ala Phe Thr Gly Pro Trp Gly Arg Pro Gln Arg Asp Gly
        115                 120                 125


Pro Ala Leu Arg Ala Thr Ala Leu Ile Ala Tyr Ala Asn Tyr Leu Ile
    130                 135                 140


Asp Asn Gly Glu Ala Ser Thr Ala Asp Glu Ile Ile Trp Pro Ile Val
145                 150                 155                 160


Gln Asn Asp Leu Ser Tyr Ile Thr Gln Tyr Trp Asn Ser Ser Thr Phe
                165                 170                 175


Asp Leu Trp Glu Glu Val Glu Gly Ser Ser Phe Phe Thr Thr Ala Val

            180                 185                 190

Gln His Arg Ala Leu Val Glu Gly Asn Ala Leu Ala Thr Arg Leu Asn
        195                 200                 205

His Thr Cys Ser Asn Cys Val Ser Gln Ala Pro Gln Val Leu Cys Phe
    210                 215                 220

Leu Gln Ser Tyr Trp Thr Gly Ser Tyr Val Leu Ala Asn Phe Gly Gly
225                 230                 235                 240

Ser Gly Arg Ser Gly Lys Asp Val Asn Ser Ile Leu Gly Ser Ile His
                245                 250                 255

Thr Phe Asp Pro Ala Gly Gly Cys Asp Asp Ser Thr Phe Gln Pro Cys
            260                 265                 270

Ser Ala Arg Ala Leu Ala Asn His Lys Val Val Thr Asp Ser Phe Arg
        275                 280                 285

Ser Ile Tyr Ala Ile Asn Ser Gly Ile Ala Glu Gly Ser Ala Val Ala
    290                 295                 300

Val Gly Arg Tyr Pro Glu Asp Val Tyr Gln Gly Gly Asn Pro Trp Tyr
305                 310                 315                 320

Leu Ala Thr Ala Ala Ala Ala Glu Gln Leu Tyr Asp Ala Ile Tyr Gln
                325                 330                 335

Trp Lys Lys Ile Gly Ser Ile Ser Ile Thr Asp Val Ser Leu Pro Phe
            340                 345                 350

Phe Gln Asp Ile Tyr Pro Ser Ala Ala Val Gly Thr Tyr Asn Ser Gly
        355                 360                 365

Ser Thr Thr Phe Asn Asp Ile Ile Ser Ala Val Gln Thr Tyr Gly Asp
    370                 375                 380

Gly Tyr Leu Ser Ile Val Glu Lys Tyr Thr Pro Ser Asp Gly Ser Leu
385                 390                 395                 400

Thr Glu Gln Phe Ser Arg Thr Asp Gly Thr Pro Leu Ser Ala Ser Ala
                405                 410                 415

Leu Thr Trp Ser Tyr Ala Ser Leu Leu Thr Ala Ser Ala Arg Arg Gln
            420                 425                 430

Ser Val Val Pro Ala Ser Trp Gly Glu Ser Ser Ala Ser Ser Val Pro
        435                 440                 445

Ala Val Cys Ser Ala Thr Ser Ala Thr Gly Pro Tyr Ser Thr Ala Thr
    450                 455                 460

Asn Thr Val Trp Pro Ser Ser Gly Ser Gly Ser Ser Thr Thr Thr Ser
465                 470                 475                 480

Ser Ala Pro Cys Thr Thr Pro Thr Ser Val Ala Val Thr Phe Asp Glu
                485                 490                 495

Ile Val Ser Thr Ser Tyr Gly Glu Thr Ile Tyr Leu Ala Gly Ser Ile
            500                 505                 510

Pro Glu Leu Gly Asn Trp Ser Thr Ala Ser Ala Ile Pro Leu Arg Ala
        515                 520                 525

Asp Ala Tyr Thr Asn Ser Asn Pro Leu Trp Tyr Val Thr Val Asn Leu
    530                 535                 540

Pro Pro Gly Thr Ser Phe Glu Tyr Lys Phe Phe Lys Asn Gln Thr Asp
545                 550                 555                 560

Gly Thr Ile Val Trp Glu Asp Asp Pro Asn Arg Ser Tyr Thr Val Pro
                565                 570                 575

Ala Tyr Cys Gly Gln Thr Thr Ala Ile Leu Asp Asp Ser Trp Gln
            580                 585                 590